# EUROPÄISCHE PATENTANMELDUNG

(11) **EP 0 836 844 A2**
(43) Veröffentlichungstag der Anmeldung: **22.04.1998**
(21) Anmeldenummer: 97117766.2
(22) Anmeldetag: 14.10.1997
(51) Int. Cl.: A61G 5/10, A61G 7/02

(54) **Hosenhalter**

(30) Priorität: 15.10.1996 DE 29617875 U
(71) Anmelder: Diekmann, Holger, 63500 Seligenstadt (DE); Trassl, Alfred, 64287 Darmstadt (DE)
(72) Erfinder: Diekmann, Holger, 63500 Seligenstadt (DE); Trassl, Alfred, 64287 Darmstadt (DE)
(74) Vertreter: Wolf, Günter, Dipl.-Ing.

(57) **Zusammenfassung**

Die Erfindung betrifft einen Hosenhalter für an Rollstühle gefesselte Personen und ist dabei insbesondere bestimmt als Urinierhilfsmittel für männliche Personen. Der Erfindung liegt die Aufgabe zugrunde, ein Vorrichtung zu schaffen, mit deren Hilfe die Hose der im Rollstuhl sitzenden Person zurückgezogen und niedergehalten werden kann und dem zufolge die betreffende Person (eine oder) beide Hände für die folgenden Erfordernisse zur Verfügung stehen. Erfindungsgemäß ist diese Aufgabe daduruch gelöst, daß der Hosenhalter (1) aus einem, in Seitenansicht gesehen, im wesentlichen C-förmigen, zwei Schenkel (2', 2'') aufweisenden Bügel (2) gebildet und dessen einer Schenkel (2') mit einem Hakenelement (3) versehen bzw. hakenförmig ausgebildet ist, wobei die Hakenöffnung (3') gegen den Innenraum (4) des Bügels (2) weist.

## Beschreibung

Die Erfindung betrifft einen Hosenhalter für an Rollstühle gefesselte Personen, wobei dieser Hosenhalter insbesondere als Urinierhilfsmittel für männliche Personen bestimmt ist.

Für an Rollstühle gefesselte Personen ist die Benutzung der Toilette meist mit beschwerlichen Maßnahmen wie z.B. das Umheben vom Rollstuhl auf die Toilette verbunden. Um hier Erleichterung zu schaffen, verwenden männliche Personen häufig zum Urinieren einen Katheter oder einen kondomartigen Überzieher mit Abflußschlauch und angeschlossenem Auffangbeutel. Diese Hilfsmittel werden, je nach Behinderung, sowohl liegend als auch sitzend angelegt. Auf jeden Fall ergibt sich das Problem, daß nach dem Öffnen der Hose diese mit der einen Hand zurückgehalten werden muß, während die andere Hand alle weiteren Erfordernisse, für die an sich zwei Hände benötigt werden, auszuführen hat. Dies erfordert von der behinderten Person hohe Geschicklichkeit und ist praktisch unmöglich von einer Person zu bewältigen, die aufgrund ihrer Behinderung gar nur noch eine Hand zur Verfügung hat.

Der Erfindung liegt die Aufgabe zugrunde, ein Vorrichtung zu schaffen, mit deren Hilfe die Hose der im Rollstuhl sitzenden Person zurückgezogen und niedergehalten werden kann und der zufolge dieser Person (eine oder) beide Hände für die folgenden Erfordernisse voll zur Verfügung stehen.

Diese Aufgabe ist mit einem Hosenhalter nach der Erfindung dadurch gelöst, daß der Hosenhalter aus einem, in Seitenansicht gesehen, im wesentlichen C-förmigen, zwei Schenkel aufweisenden Bügel gebildet und dessen einer Schenkel mit einem Hakenelement versehen bzw. hakenförmig ausgebildet ist, wobei die Hakenöffnung gegen den Innenraum des Bügels weist.

Die Handhabung des Hosenhalters geschieht dabei wie folgt: Zunächst wird das eine Schenkelende unter der Rollstuhlsitzflächenvorderkante und anschließend die Hose in das gegen den Innenraum des Bügels weisende hakenförmige Ende des Hosenhalters eingehängt oder umgekehrt,d.h., der Bügel wird zuerst in den Hosenbund eingehängt und dann an der Sitzflächenvorderkante eingehakt. Die Spannkraft des Hosengummis sorgt dabei dafür, daß sich der zwischen Rollstuhl und Hose verspannte Hosenhalter nicht von alleine lösen kann.

Um den Hosenhalter universell, d.h. auch für unterschiedlich große Personen verwenden zu können, ist es vorteilhaft, daß mindestens einer der Schenkel und/oder das Basisteil des Bügels längenveränderlich ausgebildet ist. Diese Einstellbarkeit ist weiterhin auch dann vorteilhaft, wenn die von der Person getragenen Hosen unterschiedlich dehnbar ausfallen.

Eine weitere vorteilhafte Ausführungsform besteht darin, daß der dem hakenartigen Schenkel gegenüberliegende Schenkel Mittel zur starren oder drehbaren Befestigung an einemm Rollstuhl aufweist. Diese Mittel, die noch näher erläutert werden, dienen dazu, den Hosenhalter dauerhaft am Rollstuhl zu befestigen, um ihn stets verfügbar zu haben. Der Hosenhalter ist in dieser Ausführungsform also als Zubehörteil zum Rollstuhl zu verstehen, das im Bedarfsfall bspw. einfach ausgeschwenkt wird.

Soll der Hosenhalter nicht mit dem Rollstuhl verbunden und eventuell auch im Bett verwendbar sein, so ist es vorteilhaft, daß am dem hakenartigen Schenkel gegenüberliegenden Schenkel ein plattenförmiges, sich im wesentlichen senkrecht zur Verbindungsachse zwischen den beiden Schenkeln erstreckendes Element angeordnet ist. Dieses plattenförmige Element wird im Bedarfsfall unter die Beine geklemmt (für die Klemmwirkung genügt schon das Eigengewicht der Beine) und anschließend kann die Hose, wie bisher auch schon, in das hakenförmige Ende des Hosenhalters eingehängt werden. Bei dieser Ausführungsform wird also das Widerlager für den Hosenhalter nicht mit der Sitzvorderkante des Rollstuhls sondern von den Beinen des Behinderten gebildet. Allgemein gilt also, daß der Hosenhalter entweder gegen den Behinderten selbst oder gegen einen ihm unmittelbar benachbart befindlichen Gegenstand (Bett, Rollstuhl oder dgl.) verspannbar ausgebildet sein kann.

Eine andere Lösung der Aufgabe besteht darin, daß der Hosenhalter stangenförmig und im wesentlichen geradlinig ausgebildet ist und dabei etwa auf seiner halber Erstreckungslänge ein Hakenelement aufweist.

Die Handhabung dieser kleiderbügelartigen Konstruktion ist dabei die Folgende: Zunächst legt der Behinderte den Hosenhalter auf seinen Beinen im Bereich vor den Armstützen ab, wobei die Breite des Hosenhalters so bemessen ist, daß seine Enden die Armstutzen auf beiden Seiten des Rollstuhles jeweils geringfügig überragen. Als nächstes wird dann die Hose geöffnet und in den mittig am Hosenhalter angeordneten Haken eingehängt, wobei sich die Enden des Hosenhalters dann gegen die Armstützen des Rollstuhles abstützen.

Da bei der oben beschriebenen Ausführungsform ein Verrutschen des Hosenhalters nicht mit Sicherheit auszuschließen ist, ist es vorteilhaft, daß der stangenförmige Hosenhalter an seinen beiden Enden noch näher zu erläuternde Mittel zur Befestigung am Rollstuhl aufweist.

Der erfindungsgemäße Hosenhalter und dessen vorteilhafte Weiterbildungen nach den abhängigen Ansprüchen werden nachfolgend anhand der zeichnerischen Darstellung von Ausführungsbeispielen näher erläutert.

Es zeigt:
- Fig. 1: den Hosenhalter mit Längenverstellung in Seitenansicht;
- Fig. 2: perspektivisch den Hosenhalter in einer anderen Ausführungsform;
- Fig. 3: perspektivisch eine weitere Ausführungsform des Hosenhalters;
- Fig. 4: in Seitenansicht den Hosenhalter in besonderer und bevorzugter Ausführungsform und
- Fig. 5: einen Schnitt längs Linie V-V durch den Hosenhalter gemäß Fig. 4.

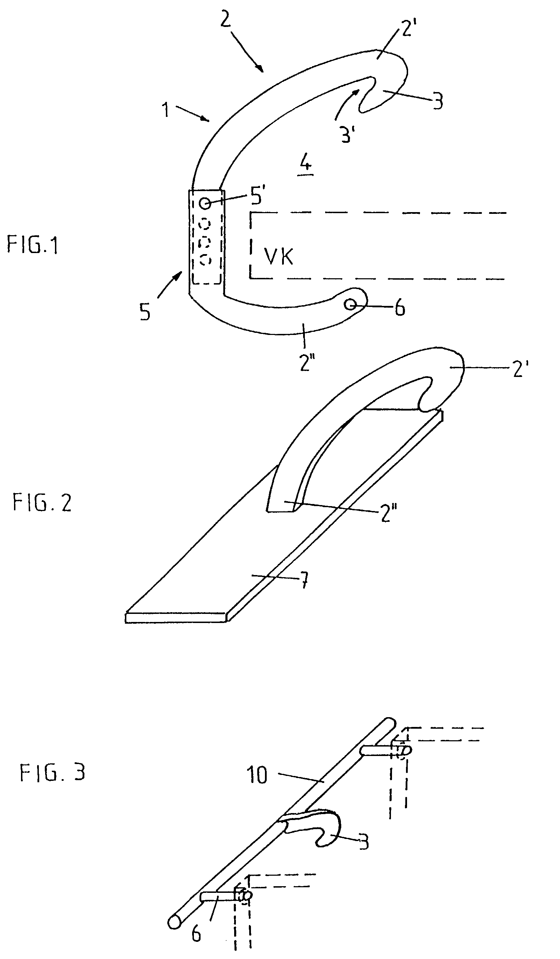

Für den Hosenhalter 1 in Fig. 1, der von an Rollstühle gefesselten Personen und insbesondere als Urinierhilfsmittel für männlichen Personen bestimmt ist, ist wesentlich, daß dieser in Seitenansicht gesehen, im wesentlichen C-förmigen, zwei Schenkel 2', 2'' aufweisenden Bügel 2 gebildet und dessen einer Schenkel 2' mit einem Hakenelement 3 versehen bzw. hakenförmig ausgebildet ist, wobei die Hakenöffnung gegen den Innenraum 4 des Bügels 2 weist.

Mit der Ausführungsform nach Fig. 1 wird das Schenkelende 2'' des Bügels 2 unter die Rollstuhlsitzflächenvorderkante VK eingehängt (gestrichelt eingezeichnet) und nach dem Einhaken der Hose in das hakenförmig ausgebildete andere Schenkelende 2' mit dieser verspannt oder , wie erwähnt umgekehrt.

In Fig. 1 ist mit dargestellt, daß das Basisteil 5 des Bügels 2 längenveränderlich ausgebildet ist. Hierbei handelt es sich bspw. um eine teleskopartige Konstruktion, bei der nach der Einstellung ein Bolzen 5' zur Fixierung dient. Wie oben erwähnt, ist diese Einstellbarkeit des Hosenhalters insbesondere deshalb vorteilhaft, weil auf diese Weise der Hosenhalter universell von unterschiedlich große Personen verwendet werden kann.

Am unteren Schenkelende 2'' des Hosenhalters 1 ist in Fig. 1 eine Bohrung 6 dargestellt. Diese dient dazu, den Hosenhalter mit Hilfe einer Schraube oder dgl. am Rollstuhl schwenkbar zu befestigen, um ihn im Bedarfsfall ausschwenken zu können und immer verfügbar zu haben.

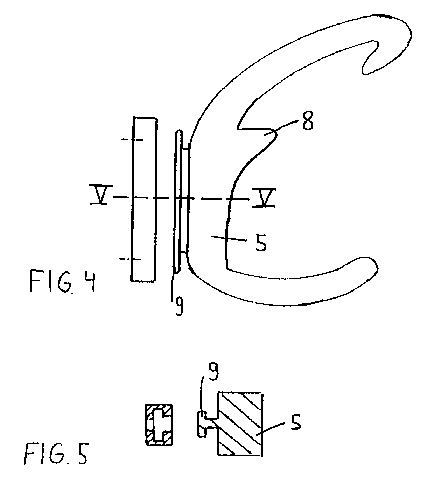

Bevorzugt wird die Ausführungsform nach Fig. 4 und 5, die innen am Basissteg 5 einen Handhabungsvorsprung 8 in der bspw. dargdestellten Form aufweist. Mit einem solchen Vorsprung 8 wird die Handhabbarkeit verbessert, und zwar mit Rücksicht auf solche Behinderungsfälle, bei denen auch die Fingerbeweglichkeit beschränkt ist. Hierbei kann bspw. der Daumen-Zeigefingerzwickel am Vorsprung 8 zum Ansatz gebracht und damit der Bügel 2 geführt werden. Ferner ist auf der anderen Seite, also außen am Bügel bzw. am Basisteil 5 eine Schiebeführung 9 mit einem darauf aufschiebbaren Wandhalter 10 angeordnet, d.h., wenn dieser Wandhalter an einer zur Toilette benachbarten Wand befestigt ist, kann der Bügel 2 bzw. der Hosenhalter leicht noch oben herausgezogen, benutzt und wieder zurückgesteckt werden und steht immer dort zur Verfügung, wo er benötigt wird. Abgesehen davon hat die Schiebeführung 9, da sie ebenfalls gewissermaßen einen äußeren Haken bildet, vorteilhaft eine weitere Funktion, nämlich die einer Aufhängehilfe für einen am Katheter befindlichen Urinsammelbeutel.

In Fig. 2 ist ein plattenförmiges, sich im wesentlichen senkrecht zur Verbindungsachse zwischen den beiden Schenkeln 2' und 2'' erstreckendes und am Schenkelende 2'' angeordnetes Element 7 dargestellt. Dieses Element 7 bzw. diese Platte ermöglichen es vorteilhafterweise, wie oben bereits erwähnt, daß der Hosenhalter auch bspw. im Bett ohne eine Verspannung gegen einen der den Behinderten umgebenden Gegenstände (Rollstuhl oder dgl.) verwendet werden kann.

Eine andere, die unabhängige Lösung darstellende Ausführungsform des Hosenhalters ist in Fig. 3 dargestellt. Bei dieser Ausführung ist wesentlich, daß der Hosenhalter 10 stangenförmig und im wesentlichen geradlinig ausgebildet ist und dabei etwa auf seiner halber Erstreckungslänge ein Hakenelement 30 aufweist. Wie schon oben erwähnt, legt bei dieser Ausführungsform der Behinderte den Hosenhalter 10 auf seinen Beinen ab und hängt seine Hose am Hakenelement 30 ein. Dadurch wird gleichzeitig der, aufgrund seiner Breite über die Armstützen des Rollstuhles AS (gestrichelt eingezeichnet) seitlich hinausragende Hosenhalter 10 gegen diese gezogen.

Um dabei eine rutschsichere Befestigung des Hosenhalters 10 an den Armstützen AS zu erreichen, ist es vorteilhaft, daß der stangenförmige Hosenhalter 10 an seinen beiden Enden 10', 10'' Mittel 6 zu Befestigung am Rollstuhl aufweist. Wie aus Fig. 3 ersichtlich sind dazu bspw. sehr einfach anzubringende Bolzen mit im Rollstuhl dafür vorgesehenen Aufnahmeöffnungen geeignet, um eine solche Fixierung zu gewährleisten.

## Patentansprüche

1. Hosenhalter für an Rollstühle gefesselte Personen und dabei insbesondere bestimmt als Urinierhilfsmittel für männliche Personen,
**dadurch gekennzeichnet,**
daß der Hosenhalter (1) aus einem, in Seitenansicht gesehen, im wesentlichen C-förmigen, zwei Schenkel (2', 2'') aufweisenden Bügel (2) gebildet und dessen einer Schenkel (2') mit einem Hakenelement (3) versehen bzw. hakenförmig ausgebildet ist, wobei die Hakenöffnung (3') gegen den Innenraum (4) des Bügels (2) weist.

2. Hosenhalter nach Anspruch 1,
**dadurch gekennzeichnet,**
daß mindestens einer der Schenkel (2', 2'') und/oder das Basisteil (5) des Bügels (2) längenveränderlich ausgebildet ist.

3. Hosenhalter nach Anspruch 1 oder 2,
**dadurch gekennzeichnet,**
daß der dem hakenartigen Schenkel (2') gegenüberliegende Schenkel (2'') Mittel (6) zur starren oder drehbaren Befestigung am Rollstuhl aufweist.

4. Hosenhalter nach einem der Ansprüche 1 bis 3,
**dadurch gekennzeichnet,**
daß am dem hakenartigen Schenkel (2') gegenüberliegenden Schenkel (2'') ein plattenförmiges, sich im wesentlichen senkrecht zur Verbindungsachse zwischen den beiden Schenkeln (2', 2'') erstreckendes Element (7) angeordnet ist.

5. Hosenhalter nach Anspruch 1 oder 2,
**dadurch gekennzeichnet,**
daß innen am Basisteil (5) des Bügels (2) ein Handhabungsvorsprung (8) angeordnet ist.

6. Hosenhalter nach einem der Ansprüche 1, 2 oder 5,
**dadurch gekennzeichnet,**
daß außen am Basisteil (5) eine Schiebeführung (9) mit einem darauf aufschiebbaren Wandhalter (10) angeordnet ist.

7. Hosenhalter für an Rollstühle gefesselte Personen und dabei insbesondere bestimmt als Urinierhilfsmittel für männliche Personen,
**dadurch gekennzeichnet,**
daß der Hosenhalter (10) stangenförmig und im wesentlichen geradlinig ausgebildet ist und dabei etwa auf seiner halber Erstreckungslänge ein Hakenelement (30) aufweist.

8. Hosenhalter nach Anspruch 7,
**dadurch gekennzeichnet,**
daß der stangenförmige Hosenhalter (10) an seinen beiden Enden (10', 10'') Mittel (60) zu Befestigung am Rollstuhl aufweist.
